# EUROPEAN PATENT SPECIFICATION

(11) **EP 0 661 048 B1**
(45) Date of publication and mention of the grant of the patent: **27.12.2000**
(21) Application number: 94909125.0
(22) Date of filing: 04.03.1994
(51) Int. Cl.: A61K 31/16, A61K 31/14

(54) **ANTIVIRAL COMPOSITIONS CONTAINING A COMBINATION OF AN IODIDE OF DIMETHYLBENZYLALKYLAMMONIUM WITH AN IODIDE OF A QUATERNARY AMMONIUM AMIDE**
ANTIVIRALE ZUSAMMENSETZUNGEN, DIE EINE KOMBINATION EINES DIMETHYLBENZYLALKYLAMMONIUMIODIDES MIT EINEM AMIDOALKYLAMMONIUMIODID ENTHALTEN
COMPOSITIONS ANTIVIRALES CONTENANT UNE COMBINAISON D'UN IODURE DE DIMETHYLBENZYLALKYLAMMONIUM ET D'UN IODURE D'UNE AMIDE D'AMMONIUM QUATERNAIRE

(30) Priority: 05.03.1993 ES 9300452
(43) Date of publication of application: 05.07.1995
(73) Proprietor: Garcia Nunez, Maria Rosalia, E-28924 Alcorcon (Madrid) (ES)
(72) Inventor: Garcia Nunez, Maria Rosalia, E-28924 Alcorcon (Madrid) (ES)
(74) Representative: Perez Bonal, Bernardo
(86) International application number: ES9400022
(87) International publication number: WO9420084

(56) References cited:
- EP-A- 0 308 564
- EP-A- 0 338 515
- WO-A-93/00813
- STN INTERNATIONAL, KARLSRUHE, XP002152158 FILE CA, CHEMICAL ABSTRACTS. AN=CA120(15):185407a. B. HUANG, 'Fast-acting virucides for Aids virus. abstract & CN 1 074 800 A (IBID.) 4 August 1993 (1993-08-04)

## Description

### SCOPE OF THE INVENTION

In general terms, the invention refers to the treatment of viral diseases. More specifically, the invention provides antiviral compositions able to reduce or even prevent the infectious effects of the Human Immunodeficiency Virus (HIV), which is the etiological agent of the Acquired Immunodeficiency Syndrome (AIDS). Therefore, they can be used to formulate pharmaceutical compounds very useful for the treatment of this disease.

### BACKGROUND OF THE INVENTION

The Human Immunodeficiency Virus (HIV) is the acknowledged agent causing the Acquired Immunodeficiency Syndrome (AIDS). The HIV belongs to the group of viruses known as retroviruses, which contain ribonucleic acid (RNA) as their genetic material and the enzyme reverse transcriptase (RT) which converts the viral RNA into DNA (desoxyribonucleic acid).

The HIV virion encompasses a membrane formed by two lipidic layers coming from the outer membrane of the host cell. Two glycoproteins protude from such membrane; they are formed by two components: gp41 (which crosses through the membrane) and gp120 (which protrudes from the membrane). The cover formed by the membrane and the glycoproteins encompasses the "nucleus" of the virion, formed by the proteins P24 (or P25) and P18. Such "nucleus" also houses the viral RNA and the RT [Gallo, R.C., Libros de Investigación y Ciencia, "EL SIDA" 1ª Ed. (1989), pp. 31-34, Editorial Prensa Científica S.A.]. The HIV genome contains at least 9 genes surrounded by the long terminal repetitions (LTR), areas which are not protein-encoded, but which serve to initiate other viral genes [Haseltine W.A. et al., Libros de Investigación y Ciencia, "EL SIDA" 1ª Ed. (1989), pp. 42-51, Editorial Prensa Científica S.A.]. The HIV joins the T4 lymphocytes, since the CD4 protein, located on the surface of the T4 lymphocytes, serves as a receiver or joining mean of the HIV [Dalgleish A.G. et al. Nature 312:763-767 (1985)]. Additionally, the HIV can also combine with macrophages [Gallo, R. C., cfr. supra].

The life cycle of the HIV requires that the virus penetrates the host patient through sexual intercourse or through blood transfusion, or even from the mother to the child. Subsequently, the virus cover integrates with the membrane of the lymphocyte, releasing the content of the virion in the cytoplasm, where the RT synthesises a DNA chain complementary to the viral RNA chain. Afterwards, and as long as the RNA degrades a second DNA chain is elaborated, and this 2-chain DNA migrates to the nucleus of the host cell, adopts a circular structure and integrates randomly at the chromosomes of the host cell. Then, the viral DNA is converted into RNA which is converted in turn into proteins. The synthesised proteins and the viral RNA combine to form a new virion which, after incorporating lipidic material from the outer cell membrane, goes out to an extra-cell medium [Gallo, R. C., cfr. supra, pp. 18-29].

AIDS is a disease characterized because the number of coadjutant/inductor T cells (CD4-) decreases inexorably. The pathology of AIDS can be detected, among other causes by the loss of spreading response of in vitro T cells, an excessive production of immunoglobulin by B cells and loss of response on the part of cytotoxic in vitro T cells. As a consequence of these alterations, the immunologic response originated by T cells is drastically reduced, thereby occurring opportunistic diseases and/or tumours which, in most cases, will end up with the death of the AIDS patient. However, as the molecular biology of HIV - the etiological agent of AIDS - is getting better known, different lines of research have been developed, hoping to stop and to revert the progressive immunodeficiency observed in patients suffering from AIDS, and to find an anti-viral agent able to prevent the HIV replication and its pathogenicity on the cells of the immune system. Mitsuya and Broder [Nature (1987) Vol. 325, pp. 773-778] have carried out an outstanding review of the existing strategies for AIDS anti-viral therapy. More recently, González Lahoz and Adrados de Llanos [Revista Clínica Española, Vol. 190, Nº 1, June 1992] have updated a review on the therapy of AIDS and its related pathologies. Especially, these authors mentioned the anti-HIV agents, those already known and/or those which are currently subject to both in vivo and in vitro tests. There are many drugs known which have a capacity of inhibiting the HIV, although there are very few able to prove a clinical efficiency which make advisable their use with patients suffering from AIDS. Among the anti-HIV agents with a better clinical experience, it is worth mentioning azide-thymidine (zydo-vudine or AZT), didesoxynosine (ddI) didesoxycytidine (ddC), ribavirine and alpha-interferon.

The AZT, which inhibits the RT, is the most commonly used retroviral agent, although it entails a series of toxic effects which affect the marrow and hypocellularity conditions which provoke anaemia, leucopenia and neutropenia. The ddI is the first anti-retroviral which meant an alternative to the use of AZT, with less secondary effects. It is used on patients with no response towards the AZT. The ddC has a stronger in vitro antiviral effect than the AZT, although its clinical use has been very restricted due to its toxicity, since it provokes serious conditions of peripheral neuropathies. The results obtained from different tests performed with ribavarine and alpha-interferon have not been too rewarding.

Currently, some research teams are working on drugs based on soluble recombining CD4, which actuates by blocking the joining of HIV to the CD4 receiver of the T4 lymphocyte or the macrophage and prevents the generation of syncytia. This alternative presents the additional advantage that the soluble recombining CD4 is able to pass through the placenta, thereby controlling the transmission of the disease through the mother-fetal channel. The only inconvenience it presents is that its average life is very short. Another alternative useful to fight against HIV, which is currently undergoing its preliminary stages, is to combine different anti-HIV drugs acting at different levels of the life cycle of HIV.

Although there are different solutions and other alternatives have been foreseen, there is still a need to find a retroviral agent which is effective and does not present the above mentioned undesirable toxic effects. In this respect, this invention provides new compositions which have an in vitro proven strong HIV inhibiting effect.

Some of the antiviral compositions provided by this invention are already well known, since they have been described in the Spanish Patent Application No. P9101563, filed by the Applicant hereof, which describes their industrial use as disinfectant, in germicide and/or fungicide compositions and to fight against nosocomial infection in hospitals. Furthermore, the PCT patent application No. WO 93/00813, and the Application for an European Patent No. 0 534 887, both filed by the applicant of this patent application, do mention the effectiveness of such compositions against three different types of microorganisms: bacteri, yeasts and fungi (Staphylococcus aureus, Saccharomyces ellipsoideus and Penicillium crysogenum). This background does not suggest at all that such germicide compositions are able to deactivate the infection caused by the HIV.

However, it has been surprising to find that some of the above mentioned compositions are able to inhibit or deactivate the in vitro activity of HIV, as evidenced by the tests performed on MT4 cells where (i) the cytopathogenic effect on such cells when they were exposed to HIV treated with an antiviral composition of those claimed by this invention; and (ii) the amount of HIV measured on the floating foams of the cell cultures have been evaluated as the expression of the reverse transcriptase activity and to detect and measure the P25 antigens. As it is well known, P25 antigens are released when the lysis of the HIV occurs. Therefore, they can be detected by means of a complexing reaction with an anti-P25 antibody and then to evaluate the amount of viruses.

Therefore, the object of this invention is to use certain iodated solutions to inhibit or deactivate the infectious capacity of retroviruses in general, and particularly the HIV, and to cooperate in the anti-AIDS therapy.

Additionally, the invention also provides new antiviral compositions useful for the treatment of AIDS and able to counterweight the infection provoked by the HIV and other retroviruses.

EP-A-0338 515 discloses the antiviral effect of some quaternary ammonium salts, the anti-AIDS activity of benzalkonium chlorides when employed in contraceptive creams, and the use of cetylpyridinium chloride in order to inactivate AIDS-causing virus in donated blood prior of tranfusion of the blood to a receiving patient. Additionally, said EP application discloses that in order to obtain a more rapid inactivation of AIDS-causing virus in the donated blood it is required to add higher concentrations of quaternary ammonium salts, what is detrimental to living tissue and may result in toxic effects in the patient receiving the blood, or alternatively to use lower concentrations of said salts during longer incubation times.

### DETAILED DESCRIPTION OF THE INVENTION

The invention provides pharmaceutical compositions that for treating the infection caused by a retrovirus. They are especially able to reduce or deactivate the infectious power of HIV and, therefore, they can be used for AIDS treatment.

The pharmaceuticcal compositions provided by this invention are formed by:
(i) a therapeutically effective amount of an antiviral composition comprising:
   a) at least, an alkyldimethylbenzylammonium iodide of general formula (I) 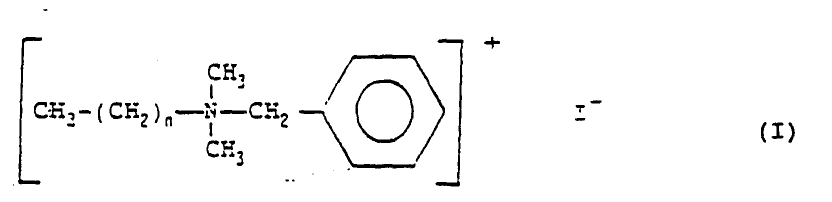 where:
      n is an integer ranging between 10 and 20, both inclusive,
      with a proportion, by weight, with respect to the whole antiviral composition, between 40% and 50%, and preferably 45%;
   b) at least, a quaternary ammonium amide iodide of general formula (II): 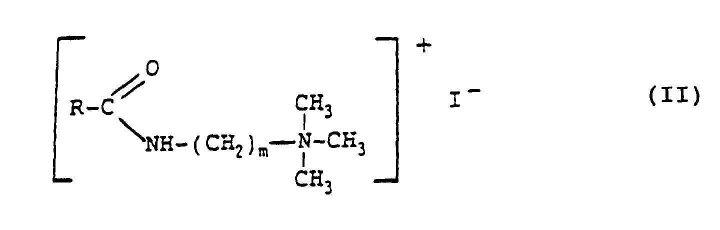 where:
      R is an alkyl radical with up to 20 carbon atoms or an alkenyl with up to 20 carbon atoms; and
      m is an integer ranging between 1 and 10, both inclusive;
      with a proportion, by weight, with respect to the whole antiviral composition, between 5% and 15%, and preferably 10%;
   c) at least, an oxyethylenated fatty alcohol with 8 moles of ethylene oxide with a proportion, by weight, with respect to the whole antiviral composition, between 5% and 15%, and preferably 10%;
   d) sublimated metallic iodine with a proportion, by weight, with respect to the whole antiviral composition, between 2% and 6%, and preferably 4%;
   e) a monoglyceride of a polyoxyethylenated fatty acid with a proportion by weight, with respect to the whole antiviral composition, between 5% and 15%, and preferably 10%;
   f) propanetriol, with a proportion by weight, with respect to the whole antiviral composition, between 3% and 7%, and preferably 5%;
   g) hexyleneglycol with a proportion, by weight, with respect to the whole antiviral composition, between 10% and 20%, and preferably 16%; and
   h) at least, an inorganic acid in an amount sufficient to set the value of the pH of the composition ranging between 4 and 6, and preferably, 4.5; and
(ii) a pharmaceutically acceptable vehicle.

The term "Alkyl group with up to 20 carbon atoms", as it is used in this description, refers to linear, cyclical or ramified chain hydrocarbon remains containing between 1 and 20 carbon atoms and presenting at least an unsaturation at any radical position.

The term "oxyethylenated fatty alcohol with 8 moles of ethylene oxide" should be understood as an alcohol with 12 to 20 carbon atoms, (both inclusive) containing 8 epoxy- groups at any position of the molecule. Among these alcohols we could mention, without limitation, those marketed by ICI, PULCRA, KAO and REWO under the trade marks Synperonic A7/A9, Pulcra KQ33/KQ20, Findet 1618/1315/20 and rewopal LA3/TA11, respectively.

Furthermore, the term "monoglyceride of a polyoxyethylenated fatty acid" should be understood as a compound formed by a molecule of glycerol with one of the hydroxy groups esterified with the remains of a fatty acid with to 12 to 20 carbon atoms and 4 to 10 oxy-ethylenated groups at any permitted position of the molecule. Among the examples of these monoglycerides we could mention without limitation the compounds marketed by KAO, REWO and PULCRA, under the trade names Findet OR/16/22, Rewoderm LI 420/ES90 and Pulcra KC11/LC10 respectively.

As far as the inorganic acid is concerned, any acid able to provide to the antiviral composition a pH value between 4 and 6 an especially 4.5 can be used. Among such acids we could mention nitric acid, sulphuric acid and others of a similar nature, except hydrochloric acid, and preferably, phosphoric acid.

As it has been previously mentioned, these compounds can be prepared according to the general procedure described in the Spanish patent application No. ES 9101563 or in the issued European patent application No. 0 534 887.

A particularly preferred antiviral composition would be as follows:

| | | |
|---|---|---|
| a) | dimethyl-estearyl benzylammonium iodide | 45% |
| b) | N-1-(3-trimethyl-ammonium)-propyl-undecylenamide iodide | 10% |
| c) | an oxyethylenated fatty alcohol with 8 moles of ethylene oxide | 10% |
| d) | sublimated metallic iodine | 4% |
| e) | a monoglyceride of a polyoxyethylenated fatty acid | 10% |
| f) | propanetriol | 5% |
| g) | Hexylenglycol | 16% |
| h) | Phosphoric acid, in a sufficient amount to set a pH value of 4.5; | |

where all the proportions are expressed in percentage terms with respect to the total weight of the antiviral composition.

With the purpose of determining whether these compositions are able to deactivate at least 10⁵ infectious HIV particles which would demonstrate its likely application to the treatment of AIDS a series of tests were performed, in order to show whether HIV Viruses treated with any of the antiviral compositions of this invention killed cells when inoculated in the appropriate cells and cultured under the most adequate conditions, and if any reverse transcriptase (RT) activity (i) and/or P25 antigen (ii) were detected on the floating foams of the cultures.

Therefore, a series of infectivity tests were performed. In their first stage, these tests basically consisted of treating HIV viruses with any of the antiviral compositions of this invention, at different concentrations and contact times. The second stage consisted of inoculating such treated HIV viruses to MT4 cells, a line of T lymphoid cells transformed with HTLV-1 (Human T-Lymphotropic Virus-1) which cause a cytopathogenic effect and the death, 8-10 days after the infection, of more than 80% when subject to a hyper-exposure to HIV.

The test carried out on MT4 cells provides a quick response, since 10 days after the cells MT4 were infected by the HIV, a cytopathogenic effect (i) could be noted and (ii) RT activity was detected on the floating foams of the cultures of infected samples.

Such cytopathogenic effect is directly related to the amount of viruses used for the infection, with the replicating effect and the expression of viral antigens by the cells. The inhibition of this effect corresponds to an inhibition of the multiplication of viruses measured by the RT activity on the floating foams of the cultures. The MT4 cells are cultured in a medium formed by RPMI 1640 (WITTAKER) supplemented by a 10% of serum of foetal veal (LABSYSTEM), 1% antibiotics (PSN GIBCO) and 1% glutamine (GIBCO).

A composition of the invention has proven to be able to deactivate or reduce the infectious effect of HIV in vitro on MT4 cells, as it can be seen in Example No. 2 attached to this descriptive report. Furthermore, it is also shown that a 1-minute treatment of the virus with one of the preferred anti-viral compounds of this invention diluted at 1% or 5% may reduce by 10⁵ times the infectious power of the HIV on altered cells with a CD4+ lymphocytic origin.

The mechanism that enables this antiviral compositions to reduce or deactivate de infectious power of the HIV is not completely known, although the results assume, as a hypothesis, that it inhibits the action of the reverse transcriptase (RT), probably by provoking a structural change in such enzyme, by changing the structure either of the polymerase or of the ribonuclease composing the RT. Provided that such assumption is certain, the compositions of this invention could be useful to reduce or inhibit the infectious power of any retrovirus, since such viruses share an RT enzyme with polymerase or ribonuclease activity. Therefore, the invention also provides antiretroviral compositions that can be used to reduce or even inhibit the infectious power of a given retrovirus.

The antiviral compositions of this invention may be used to prepare drugs and pharmaceuticals for the treatment of infections caused by retroviruses, especially the HIV, and to help in the treatment of AIDS. Such drugs or pharmaceuticals may be prepared by combining a therapeutically-effective amount of the antiviral composition with pharmaceutically acceptable appropriate vehicles.

To illustrate the execution of the invention, some examples are attached herebelow, with no limitation.

### EXAMPLES

### Example 1 Treatment of the HIV Virus

### 1.A. Characteristics of the viruses used

The HIV-1 (LAI strain) used for these examples has been supplied by the Institut Pasteur where it is produced on T-lymphoblastoid cells chronically infected (line CEM-HIV-1/LAI). The cells are centrifuged every 3 or 4 days and suspended within a fresh culture (RPMI 1640 containing a 10% of serum of foetal veal, 1% antibiotics and 1% glutamine). Then they are blended with non infected CEM cells (unmatured CD4+ lymphocytes) until they reach a final concentration of 0.5·10⁶ cells/ml to keep the production of viruses stable.

The virus present on the floating foams of the cultures concentrates by centrifugation at 17,000 R.P.M. for 3 hours and the residue is re-suspended on a NTE buffer. Then it is distributed into small volumes and stored at - 80 °C.

The HIV-1/LAI strain virus (hereinafter HIV-1) used for the following tests has the following titre:
(i) 10⁷ IU/ml (Infectious units) for MT4 cells, and
(ii) a reverse transcriptase (RT) activity of 4·10⁹.

### 1.B Characteristics of the antiviral compounds used for the treatment of the HIV-1 virus

An antiviral composition of this invention has been used for the treatment of the HIV-1 viruses of the example 1-A. It is formed by:

| | | |
|---|---|---|
| a) | dimethyl-stearyl benzylammonium iodide | 45% |
| b) | N-1-(3-trimethyl-ammonium)-propyl-undecylenamide iodide | 10% |
| c) | an oxyethylenated fatty alcohol with 8 moles of ethylene oxide* | 10% |
| d) | sublimated metallic iodine | 4% |
| e) | a monoglyceride of a polioxyethylenated fatty acid* | 10% |
| f) | Propanetriol | 5% |
| g) | Hexylenglycol | 16% |
| h) | Phosphoric acid, in a sufficient amount to set a pH value of 4.5; | |
| where all the proportions are expressed in percentage terms with respect to the total weight of the antiviral composition. | | |

| | | |
|---|---|---|
| * a combination of several of the products mentioned in the report. | | |

### 1.C Treatment of the HIV-1 with an antiviral compound

The HIV-1 virus of the example 1.A was diluted until reaching a dilution of 1/12.5 (with a theoretical activity of 0.8·10⁶ UI/ml on NT4 cells) using solutions of the antiviral composition described in the Example 1.B with concentrations of 50%, 10%, 5% and 1%. The contact times were 1 and 3 minutes, at room temperature.

The following samples of viruses treated with the antiviral composition previously described were prepared, using 80 µl of HIV-1 diluted in 1 ml of antiviral composition at different concentrations. After leaving the virus/antiviral composition mixture in contact for a period of 1 to 3 minutes (depending on the case) at room temperature, 10 ml of NTE buffer were added (except in the case of the sample NG12, where 10 ml of RPMI culture medium were used). Then, the samples prepared containing viruses and antiviral compositions were prepared indicating, on each case, the contact time and the concentration of the antiviral composition.

| Sample code | Contact time (min) | Concentration of the antiviral composition (1%) |
|---|---|---|
| NG8 | 1 | 1 |
| NG9 | 1 | 5 |
| NG10 | 1 | 10 |
| NG11 | 1 | 50 |
| NG12 | 3 | 1 |
| NG13 | 3 | 5 |
| NG14 | 3 | 10 |
| NG15 | 3 | 50 |

Following the second dilution, the samples were centrifuged during 35 minutes at 40,000 R.P.M., to concentrate the residual viruses and to eliminate the antiviral composition toxic for the infectivity test cells. After the centrifugation, the residues are collected in 0.5 ml of culture medium (RPMI), distributed in small volumes and stored at -80 °C.

Additionally, some positive and negative controls were prepared. The positive controls (viruses without antiviral composition were the following ones:
- NG1:: Formed by the HIV-1 virus diluted at 1/12.5 in a culture medium and frozen at -80 °C;
- NG2:: Formed by the HIV-1 virus diluted at 1/12.5 in a culture medium and subsequently diluted and centrifuged in the same manner as the viruses samples treated with the antiviral composition (NG8 to NG15) to evaluate the loss of infectiveness during the centrifugation stage; and
- NG3:: Formed by the HIV-1 virus diluted at 1/12.5 in a culture medium left at room temperature during the whole span of the test (3 minutes) and subsequently diluted and centrifuged in the same manner as the samples treated with the antiviral composition (NG8 to NG15) to evaluate the loss of infectiveness due to the test conditions.

The negative controls (antiviral composition without viruses) were the following ones:
- NG4:: 1 ml of antiviral composition at 1% was diluted in 10 ml of culture medium and was subsequently centrifuged under the same conditions as those described above for the viruses samples treated with the antiviral composition, with the purpose of controlling the eventual toxicity on the cells of the infectiousness test due to traces of residual antiviral composition not eliminated during the centrifugation.
- NG5:: Similar to NG4, but using an antiviral composition with a concentration of 5%;
- NG6:: Similar to NG4, but using an antiviral composition with a concentration of 10%; and
- NG7:: Similar to NG4, but using an antiviral composition with a concentration of 50%;

The cells of the infectiousness test not infected.

### Example 2 Evaluation of the residual infectious power of the treated viruses samples on MT4 cells

In order to evaluate the residual infectious power of the HIV-1 samples treated with the antiviral composition of Example 1.B on MT4 cells, the following tests were performed:
A. Assessment of the in vitro cytopathogenic effect on MT4 cells subject to a hyper-exposure to the virus;
B. Assessment of the reverse transcriptase activity (RT) present on the floating foams of the cell cultures; and
C. Detection and measure of the P25 antigen present on the floating foams of the cell cultures.

### 2.A Assessment of the in vitro cytopathogenic effect

3·10⁵ MT4 cells were placed on COSTAR plate containers with 24 receptacles, adding 0.25 ml or 0.1 ml of each sample of virus treated with the antiviral composition of Example 1.B at 1% or 5% respectively (Day "0" of the infection). The receptacles were filled with 1 ml of culture medium (RPMI 1640) and a final dilution of 1/4 and 1/10 of the samples were respectively obtained.

The virus samples treated with antiviral composition at 10% and 50% were tested similarly, but with final dilutions of 1/50 and 1/100.

The three samples of the positive witnesses (NG1, NG2 and NG3) were treated similarly, but at dilutions ranging between 10⁻⁴ and 10⁻⁶.

After 3 days (day +3 post-infection) at 37 °C, 750 µl of floating foam were replaced by fresh culture and the cells were diluted from 3 to 4 times to adjust them to a concentration of 3·10⁵ cells/ml. The day +6 (post-infection), the cells were observed through the microscope and the cytopathogenic effect and the concentration of cells were set at 3·10⁵ cells/ml again. The day +10 (post-infection) the cytopathogenic effect and the cell mortality were assessed through a microscope observation after a Congo blue tinction.

A strong toxicity was observed on MT4 cells with the 1/4 dilution of NG4 and NG5 negative witnesses (without viruses), with 1/4 and 1/10 dilutions of NG6 and with 1/4, 1/10 and 1/50 dilutions of NG7. As it is shown herebelow, only those results obtained from the samples inoculated with non-toxic dilutions for the cells -i.e., 1/10 for those treatments at 1% and 5%; 1/50 for the treatment at 10% and 1/100 for the treatment at 50%- are presented.

Table I shows the cytopathogenic effect observed in MT4 cells inoculated with the samples of witness viruses or the samples of the viruses treated with one of the antiviral compositions provided by this invention, and more specifically, that described in the Example 1.B.

**TABLE I**

| | | | |
|---|---|---|---|
| HIV-1 infectiousness treated or not with an antiviral composition for the cell line MT4: | | | |

| Analysis of the cytopathogenic effect after 10 days of the infection. | | | |
|---|---|---|---|
| | SAMPLE | DILUTION | ECP⁰ |
| A | NG1 | 10⁻⁶ | ++ |
| | NG2 | 10⁻⁶ | ++ |
| | NG3 | 10⁻⁶ | ++ |
| B | NG4 | 1/4 | |
| | | 1/10 | NT |
| | NG5 | 1/4 | |
| | | 1/10 | NT |
| | NG6 | 1/4 | |
| | | 1/10 | |
| | | 1/50 | NT |
| | NG7 | 1/4 | |
| | | 1/10 | |
| | | 1/50 | |
| | | 1/100 | NT |
| C | | | - |
| D | NG8 | 1/10 | - |
| | NG9 | 1/10 | - |
| | NG10 | 1/50 | - |
| | NG11 | 1/100 | - |
| | NG12 | 1/10 | - |
| | NG13 | 1/10 | - |
| | NG14 | 1/50 | - |
| | NG15 | 1/100 | - |

| | | | |
|---|---|---|---|
| ECP⁰: Cytopathogenic effect after ten days of the infection and Congo blue tinction: meaning: | | | |
| - Without pathogenic effect; | | | |
| + Around 70% of dead cells; and | | | |
| ++ 100% of dead cells. | | | |
| NT: Non-toxic | | | |
| A: Positive witnesses (viruses without antiviral composition) | | | |
| B. Negative witnesses (without viruses) | | | |
| C: Normal cell witness | | | |
| D: Samples of viruses treated with the antiviral composition. | | | |

In the light of the results shown in Table I, no cytopathogenic effect can be appreciated when the cells have been inoculated with dilutions at 1/10, or at 1/50 or 1/100 of the samples of viruses treated with the antiviral composition (NG8-NG15), irrespective of the concentration of the antiviral composition tested and the contact times. On the other hand, the samples of the control viruses (NG1, NG2 and NG3) provoked 100% of cell mortality 10 days after the infection, even when the inoculation of cells was performed using a higher dilution (10⁻⁶).

### 2.B Measure of the Reverse transcriptase activity (RT) at the floating foam of the cell cultures

The presence of viruses in the MT4 cells inoculated with the different samples has been carefully followed up through the measure of the RT activity at the floating foam of the cell cultures, and additionally, by measuring and detecting the P25 antigen on such foams.

The measure of the RT activity has been performed on the basis of the foams of the cell cultures previously obtained (Example 2.A) kept after each cell passage (days 3, 6 and 10) and each sample (two samples each).

The RT activity has been measured on 0.5 ml of the above mentioned floating foams, concentrated 50 times through ultra-centrifugation at 95,000 rpm for 5 minutes on a BECKMAN TL 100 device.

The residues were suspended in 10 µl of NTE and 0.1 of Triton X.100. The enzymatic activity was revealed by adding 40 µl of the following reaction mixture: Tris 50 mM pH 7.9; KCl 20 nM, MgCl₂ 5 nM, dithiotreithol 1 mM, poly rA 0.05 D.O. /ml, oligo dT₁₂₋₁₈ 0.05 D.O./ml and ³HTTP 5 µCi (specific activity: 30 Ci/mmol). After one hour at 37 °C, the non soluble products in acid medium were precipitated by adding trichloroacetic acid at 20%, filtering them with a Millipore filter 0.45 um and measuring their β radioactivity with the help of a flashing counter (BETAmatic; KONTRON).

Table II shows the results of the positive controls (NG1-NG3) assessed at different dilutions, to evaluate their infecting activity and calculate the loss of infecting power due to the treatment with the antiviral composition.

Table II shows that both receptacles are positive at 10⁻⁶, either on the 6th day (NG2) or the tenth day (NG1 and NG3). Under these testing conditions, the titre of the virus is defined as the inverse of the infectious dilution at 100% for the MT4 cells. Therefore, the titre of the NG1, NG2 and NG3 solutions equals or exceeds 10⁶ UI/ml.

**TABLE II**

| **INFECTIOUSNESS TEST ON MT4 CELLS IN VITRO** Titre of the dilutions of the virus samples not treated with the antiviral composition (positive witnesses) | | | | | |
|---|---|---|---|---|---|
| | | RT activity in cpm/0.5 ml* | | | |
| Sample | Dilution | +3 | +6 | +10 | ECP⁰ |
| NG1 | 10⁻⁴ | 36153 | 24457 | 768 | ++ |
| | | 55791 | 32757 | 1497 | ++ |
| | 10⁻⁵ | 16146 | 21793 | 1862 | ++ |
| | | 10791 | 27784 | 2539 | ++ |
| | 10⁻⁶ | 2231 | 2804 | 5128 | ++ |
| | | 1889 | 5249 | 8676 | ++ |
| NG2 | 10⁻⁴ | 5627 | 13350 | 6880 | ++ |
| | | 4723 | 9654 | 3011 | ++ |
| | 10⁻⁵ | 1870 | 20737 | 7645 | ++ |
| | | 1138 | 9636 | 3670 | ++ |
| | 10⁻⁶ | 2346 | 12880 | 9272 | ++ |
| | | 895 | 11529 | 3781 | ++ |
| NG3 | 10⁻⁴ | 7895 | 8794 | 1984 | ++ |
| | | 10870 | 9908 | 3515 | ++ |
| | 10⁻⁵ | 1960 | 23144 | 5305 | ++ |
| | | 2993 | 18267 | 5723 | ++ |
| | 10⁻⁶ | 1228 | 2118 | 8703 | ++ |
| | | 1596 | 16678 | 118749 | ++ |
| Single Cells | | 369 | 620 | 673 | - |
| | | 668 | 508 | 711 | - |

| | | | | | |
|---|---|---|---|---|---|
| ECP⁰: Cytopathogenic effect after ten days of the infection and Congo blue tinction: meaning: | | | | | |
| - Without cytopathogenic effect; | | | | | |
| + Around 70% of dead cells; and | | | | | |
| ++ 100% of dead cells. | | | | | |
| (*) Virus activity has been calculated by means of the PT activity on 0.5 ml of floating foams of the culture after 3, 6 and 10 days of the infection. It is considered to be positive when the RT activity exceeds **5000 cpm/0,5 ml** | | | | | |

Table III shows the results of virus RT activity at the floating foams of the cell cultures prepared by inoculating MT4 cells with the samples of virus treated with the antiviral composition (NG8 to NG15). Table III also shows that all the floating foams of the cells inoculated with the samples of the viruses treated present a negative RT activity, irrespective of the concentration of the composition and the contact time.

**TABLE III**

| **INFECTIOUSNESS OF HIV-1 TREATED FOR THE MT4 CELL LINE** Titre of the RT viral activity at the floating foams | | | | | |
|---|---|---|---|---|---|
| | | RT activity in cpm/0.5 ml* | | | |
| Sample | Dilution | +3 | +6 | +10 | ECP⁰ |
| NG8 | 1/10 | 735 | 1406 | 234 | - |
| | | 921 | 742 | 468 | - |
| NG12 | 1/10 | 1471 | 1142 | 720 | - |
| | | 1855 | 563 | 558 | - |
| NG9 | 1/10 | 700 | 552 | 417 | - |
| | | 366 | 675 | 375 | - |
| N13 | 1/10 | 403 | 732 | 948 | - |
| | | 483 | 387 | 1337 | - |
| NG10 | 1/50 | 391 | 462 | 927 | - |
| | | 334 | 206 | 1120 | - |
| NG14 | 1/50 | 447 | 239 | 286 | - |
| | | 505 | 246 | 269 | - |
| G11 | 1/100 | 476 | 202 | 420 | - |
| | | 689 | 285 | 688 | - |
| NG15 | 1/100 | 122 | 233 | 55 | - |
| | | 466 | 189 | 353 | - |
| Single cells | | 787 | 718 | 822 | - |
| | | 616 | 292 | 356 | - |

| | | | | | |
|---|---|---|---|---|---|
| ECP⁰: Cytopathogenic effect after ten days of the infection and Congo blue tinction: meaning: | | | | | |
| - Without cytopathogenic effect; | | | | | |
| + Around 70% of dead cells; and | | | | | |
| ++ 100% of dead cells. | | | | | |
| (*) Virus activity has been calculated by means of the RT activity on 0.5 ml of floating foams of the culture after 3, 6 and 10 days of the infection. It is considered to be positive when the RT activity exceeds **5000 cpm/0,5 ml** | | | | | |

In the light of the negative results of the cytopathogenic effect and the extra-cellular viral activity detected by means of enzymatic measure, it can be concluded that the composition tested is efficient to deactivate the infectious power of the HIV-1 virus.

### 2.C Detection of the P25 antigen of HIV-1 at the floating foams of the cell culture

As it has been previously mentioned, the virus activity in MT4 cells inoculated with the treated virus samples has been followed up, both by the RT activity and the measure of the P25 antigen present at the floating foams of the cell cultures kept after each passage.

The measure of the P25 antigen has been performed on the basis of the floating foams present at the cultures inoculated with the viruses treated with the antiviral composition described in Example 1.B at different intervals and concentrations (Samples NG8 to NG15) and from the floating foams of the cultures of cells infected with positive witness viruses (Samples NG1, NG2 and NG3) kept after each passage (after 3, 6 and 10 days).

A very sensible immunoenzymatic technique has been applied to confirm the results obtained through the measure of the RT activity on the floating foams. For this purpose, the kit ELAVIA AgI, marketed by the Société Diagnostics Pasteur has been used. It enables to detect from 5 to 10 picograms of P25 antigen per ml.

The p25 antigen released after the lysis of the virus and eventually present at the tested samples is combined with an anti-P25 monoclonal antibody immobilised in the receptacles of the microplates. Then, the antigen is revealed by adding biotin-marked anti-P25 polyclonal antibodies which are recognized by a streptavidin-peroxidase conjugated. The presence of this complex is revealed by means of an enzymatic reaction, after adding o-phenylindamine. The product obtained is tincted and it can be subsequently revealed by measuring the optical density at 492 nm. The amount of product formed during the enzymatic reaction is in direct ratio to the amount of antigen present on the sample tested.

As it can be appreciated (Example 2.B) the floating foams used to assess the RT activity offered negative results. However, it could be noticed that those floating foams with negative results of RT activity contain P25 antigen. However, it can be appreciated that the amount of P25 antigen decreases with the number of cell passages, which suggests that the presence of residual viral antigen corresponding to the inoculum is progressively eliminated in the course of such cell passages. In the light of the absence of such cytopathogenic effect and the absence of retroviral enzymatic activity at the floating foams, this hypothesis seems to be feasible. The antigen detected does not correspond to an active replication of the virus treated with antiviral composition on MT4 cells.

In the light of the results obtained, and more specifically, on the basis of the absence of cytopathogenic effect on MT4 cells, the absence of extracellular virus activity detected by retroviral activity at the floating foams of the cell cultures and the decrease in the amount of P25 antigen with the increase in the number of cell passages, it can be said that the antiviral composition described in the Example 1.A and tested at 1%, 5%, 10% and 50% is efficient to deactivate the in vitro infectious power of HIV-1 on MT4 cells.

In the light of the titres of the viruses used and the dilutions employed for the infectiousness test, it can be concluded that the treatment of the virus for one minute with solutions of the antiviral composition at 1% and 5% is enough to reduce by 5 logarithmic units the infectious power of HIV-1 on altered cells with a CD4+ lymphocytic origin. Under the same conditions, the solutions at 10% and 50% deactivate at least 4 logarithmic units of infectious virus.

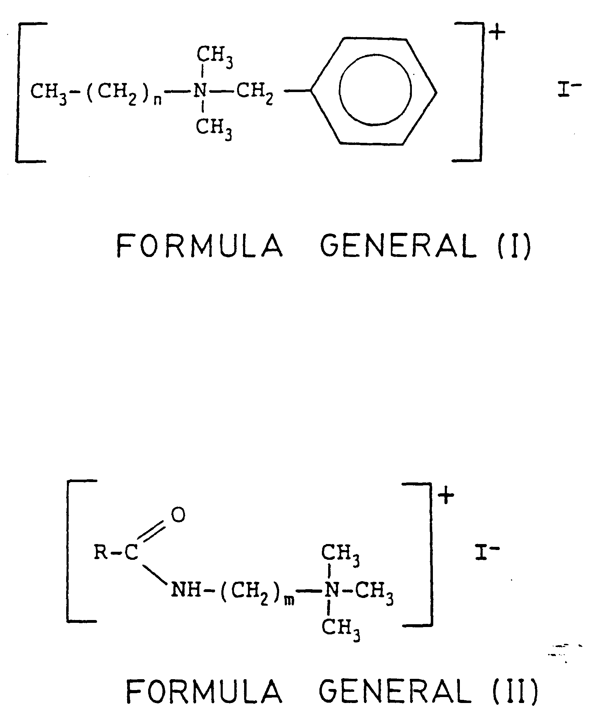

## Claims

1. A pharmaceutical composition for treating the infection caused by a retrovirus, comprising:
(i) a therapeutically effective amount of an antiviral composition comprising:
a) at least, an alkyldimethylbenzylammonium iodide of formula (I) 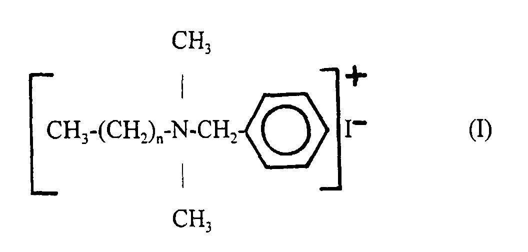 wherein n is an integer ranging between 10 and 20, both inclusive; with a proportion, by weight, with respect to the whole antiviral composition between 40% and 50%;
b) at least, a quaternary ammonium amide iodide having the general formula (II) 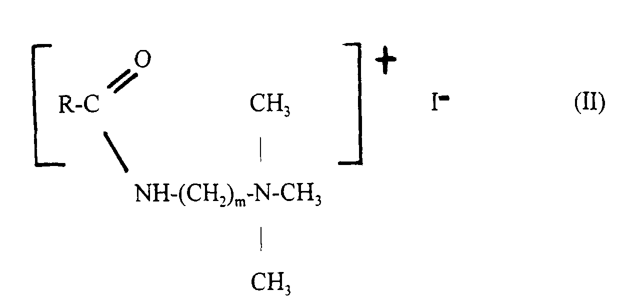 wherein R is an alkyl group having up to 20 carbon atoms or an alkenyl group having up to 20 carbon atoms; and m is an integer ranging between 1 and 10, both inclusive;
with a proportion, by weight, with respect to the whole antiviral composition between 5% and 15%;
c) at least, an oxyethylenated fatty alcohol with 8 moles of ethylene oxide, with a proportion, by weight, with respect to the whole antiviral composition between 5% and 15%;
d) sublimated metallic iodine, with a proportion, by weight, with respect to the whole antiviral composition between 2% and 6%;
e) a monoglyceride of a polyoxyethylenated fatty acid, with a proportion, by weight, with respect to the whole antiviral composition between 5% and 15%;
f) propanetriol, with a proportion, by weight, with respect to the whole antiviral composition between 3% and 7%;
g) hexylenglycol, with a proportion, by weight, with respect to the whole antiviral composition between 10% and 20%; and
h) at least, an inorganic acid in an amount sufficient to set the pH value of the composition between 4 and 6; and
(ii) a pharmaceutically acceptable vehicle.

2. Pharmaceutical composition according to claim 1, suitable for deactivating or decreasing the infectious potency of the human immunodeficiency virus (HIV).

3. Pharmaceutical composition according to claim 2, suitable for treating the Acquired Immunodeficiency Syndrome (AIDS).

4. Pharmaceutical composition according to anyone of claims 1 to 3, comprising:
(i) a therapeutically effective amount of an antiviral composition comprising:
| | | |
|---|---|---|
| a) | dimethylstearylbenzylammonium iodide | 45% |
| b) | N-1-(3-trimethylammonium)-propyl-undecylenamide iodide | 10% |
| c) | an oxyethylenated fatty alcohol with 8 moles of ethylene oxide | 10% |
| d) | sublimated metallic iodine | 4% |
| e) | a monoglyceride of a polyoxyethylenated fatty acid | 10% |
| e) | propanetriol | 5% |
| f) | hexylenglycol | 16% |
| h) | phosphoric acid in an amount sufficient to set the pH value of the composition at 4.5; | |
wherein all the proportions are expressed in percentage terms with respect to the total weight of the antiviral composition; and
(ii) a pharmaceutically acceptable vehicle.

5. Use of an antiviral composition formed by:
a) at least, an alkyldimethylbenzylammonium iodide of formula (I) 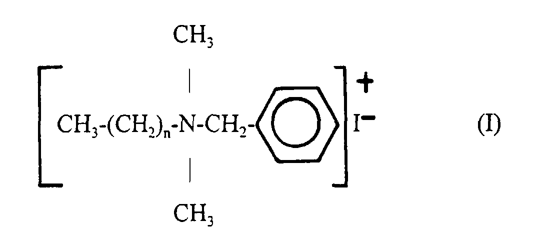 wherein n is an integer ranging between 10 and 20, both inclusive;
with a proportion, by weight, with respect to the whole antiviral composition between 40% and 50%;
b) at least, a quaternary ammonium amide iodide having the general formula (II) 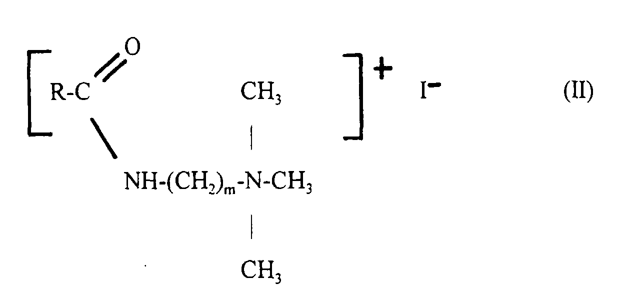 wherein R is an alkyl group having up to 20 carbon atoms or an alkenyl group having up to 20 carbon atoms; and m is an integer ranging between 1 and 10, both inclusive; with a proportion, by weight, with respect to the whole antiviral composition between 5% and 15%;
c) at least, an oxyethylenated fatty alcohol with 8 moles of ethylene oxide, with a proportion, by weight, with respect to the whole antiviral composition between 5% and 15%;
d) sublimated metallic iodine, with a proportion, by weight, with respect to the whole antiviral composition between 2% and 6%;
e) a monoglyceride of a polyoxyethylenated fatty acid, with a proportion, by weight, with respect to the whole antiviral composition between 5% and 15%;
f) propanetriol, with a proportion, by weight, with respect to the whole antiviral composition between 3% and 7%;
g) hexylenglycol, with a proportion, by weight, with respect to the whole antiviral composition between 10% and 20%; and
h) at least, an inorganic acid in an amount sufficient to set the pH value of the composition between 4 and 6;
in the preparation of a drug for the treatment of infections caused by retroviruses.

6. Use, according to claim 5, wherein said drug is suitable for deactivating or decreasing the infectious potency of the human immunodeficiency virus (HIV).

7. Use, according to claim 6, wherein said drug is suitable for treating the Acquired Immunodeficiency Syndrome (AIDS).

8. Use, according to any one of claims 5 to 7, wherein the antiviral composition is formed by:
| | | |
|---|---|---|
| a) | dimethylstearylbenzylammonium iodide | 45% |
| b) | N-1-(3-trimethylammonium)-propyl-undecylenamide iodide | 10% |
| c) | an oxyethylenated fatty alcohol with 8 moles of ethylene oxide | 10% |
| d) | sublimated metallic iodine | 4% |
| e) | a monoglyceride of a polyoxyethylenated fatty acid | 10% |
| f) | propanetriol | 5% |
| g) | hexylenglycol | 16% |
| h) | phosphoric acid in an amount sufficient to set the pH value of the composition at 4.5; | |
wherein all the proportions are expressed in percentage terms with respecto to the total weight of the antiviral composition.

## Patentansprüche

1. Ein pharmazeutisches Präparat zur Behandlung von retrovirösen Infektionen. Komposition:
(i) Eine therapeutisch wirksame Menge eines antiviralen Wirkstoffes folgender Zusammensetzung:
a) Mindestens einem Alkyl-dimethyl-benzyl-ammonium-iodid folgender Struktur (I) 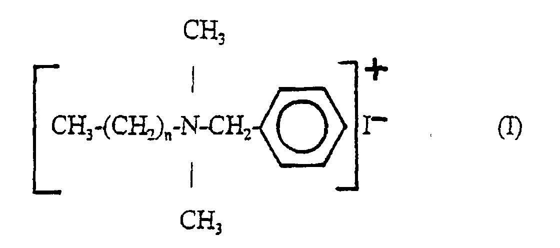 wobei n eine ganze Zahl ist, die zwischen 10 und 20 (beide inklusive) variieren kann;
Der Massenanteil beträgt, gemessen am antiviralen Gesamtpräparat, 40% bis 50%;
b) wenigstens ein Jodid eines quartären Ammonium Ions und Amids mit folgender, genereller Strukturformel (II) 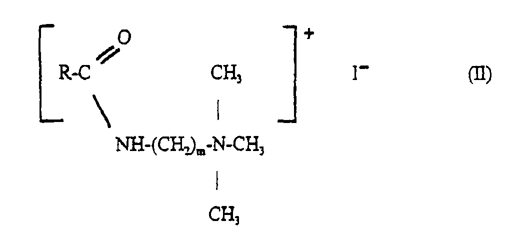 in der R ein Alkyl-Rest mit bis zu 20 Kohlenstoffatomen ist; m ist eine ganze Zahl, die von 1 bis 10 variieren kann, beide eingeschlossen;
Mit einem Massenanteil, gemessen am antiviralen Gesamtpräparat, von 5% bis 15%;
c) wenigstens ein, mit 8 mol Ethylenoxid oxiethylenisierten, Fettalkohol mit einem Massenanteil von 5 bis 15%, gemessen am antiviralen Gesamtpräparat;
d) Metallisches Jod als Sublimat, mit einem Massenanteil am antiviralen Gesamtpräparat von 2% bis 6%;
e) Ein Monoglycerid einer poly-oxiethylenisierten Fettsäure, mit einem Massenanteil von 5% bis 15%, gemessen am antiviralen Gesamtpräparat;
f) Propantriol in einem Massenanteil von 3% bis 7% des antiviralen Gesamtpräparates;
g) Hexylenglykol in einem Massenanteil von 10% bis 20%, gemessen am antiviralen Gesamtpräparat;
h) Mindestens einer organischen Säure in ausreichender Menge, um den pH-Wert des Präparates auf 4 bis 6 einstellen zu können; und
(ii) einer pharmazeutisch vertretbaren Darreichungsform.

2. Pharmazeutische Zusammensetzung gemäß den Angaben unter Klausel 1, angemessen, um das Infektionspotential des humanen Immundefizienzvirus (HIV) zu inaktivieren oder zu verringern (HIV).

3. Pharmazeutische Zusammensetzung gemäß den Angeben unter Klausel 2, angemessen, um das Syndrom der erworbenen Immundefizienz (AIDS) zu behandeln

4. Pharmazeutische Zusammensetzung gemäß einer der Klauseln 1 bis 3, welche:
(i) eine therapeutische wirksame Menge eines antiviralen Präparates enthält, welche folgende Wirkstoffe enthält:
| | | |
|---|---|---|
| a) | Dimethylesteraryl-benzyl-ammonium-jodid | 45% |
| b) | N-1-(3-Trimethylammonium) -propyl-undekylenamid-Jodid | 10% |
| c) | Einen, mit 8 mol Ethylenoxid Oxiethylenisierten, Fettalkohol | 10% |
| d) | Metallisches Jod als Sublimat | 4% |
| d) | Ein Monoglycerid einer polyoxiethylenisierten Fettsäure | 10% |
| f) | Propantriol | 5% |
| g) | Hexylenglykol | 16% |
| h) | Phosphorsäure in ausreichender Menge, um den pH-Wert des Präparates auf 4,5 einzustellen; | |
wobei sämtliche Prozentangaben sich auf den Massenanteil des Wirkstoffs am Gesamtgewicht des antiviralen Gesamtpräparats beziehen; Sowie
(ii) eine pharmazeutisch vertretbare Darreichungsform.

5. Gebrauch eines antiviralen Präparats folgender Zusammensetzung:
a) Mindestens ein Alkyl-dimethyl-benzyl-ammonium-Jodid folgender Struktur (I) 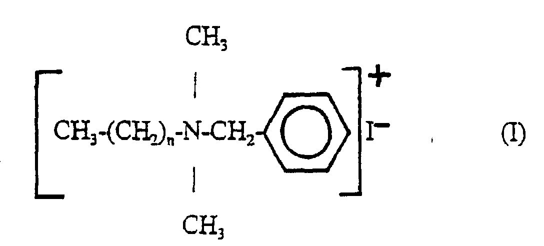 in der n eine ganze Zahl ist, deren Wert zwischen 10 und 20 (beide eingeschlossen) variieren kann;
mit einem Massenanteil am antiviralen Gesamtpräparat von 40% bis 50%;
b) wenigstens ein quartäres Ammonium Ion und Amid mit folgender genereller Strukturformel (II) 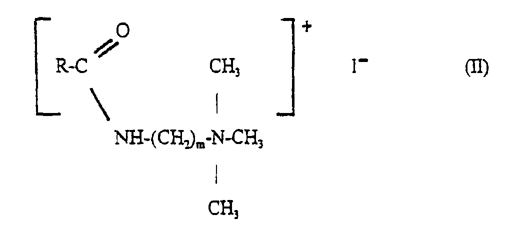 in der R ein Alkyl-Rest mit bis zu 20 Kohlenstoffatomen ist oder ein Alkenyl-Rest mit bis zu 20 Kohlenstoffatomen; m ist eine ganze Zahl, die von 1 bis 10 (beide eingeschlossen) variieren kann; Mit einem Massenanteil am antiviralen Gesamtpräparat von 5% bis 15%;
c) Wenigstens ein, mit 8 mol Ethylenoxid oxiethylenisierter, Fettalkohol mit einem Massenanteil von 5% bis 15%, gemessen am antiviralen Gesamtpräparat;
d) Metallisches Jod als Sublimat, mit einem Massenanteil am antiviralen Gesamtpräparat von 2% bis 6%;
e) Ein Monoglycerid einer poly-oxiethylenisierten Fettsäure mit einem Massenanteil von 5% bis 15%, gemessen am antiviralen Gesamtpräparat;
f) Propantriol in einem Massenanteil von 3% bis 7% des antiviralen Gesamtpräparates;
g) Hexylenglykol in einem Massenanteil von 10% bis 20%, gemessen am antiviralen Gesamtpräparat;
h) Mindestens einer organischen Säure in ausreichender Menge um den pH-Wert des Präparates auf 4 bis 6 einstellen zu können;
für die Herstellung eines Medikamentes zur Behandlung von Infektionen, die durch Retroviren hervorgerufen wurden.

6. Nutzung gemäß Klausel 5, in der das besagte Medikament für die Inaktivierung oder Verminderung des Infektionspotentials des humanen Immundefizienzvirus (HIV) geeignet ist.

7. Nutzung gemäß Klausel 6, in der das besagte Medikament für die Behandlung des Syndroms der erworbenen Immundefizienz (AIDS) geeignet ist.

8. Nutzung gemäß einer beliebigen der Klauseln 5 bis 7, in der das antivirale Präparat sich wie folgt zusammensetzt:
| | | |
|---|---|---|
| a) | Dimethylesteraryl-benzyl-ammonium-iodid | 45% |
| b) | N-1-(3-Trimethylammonium) -propyl-undekylenamid-iodid | 10% |
| c) | Ein, mit 8 mol Ethylenoxid oxiethylenisierter, Fettalkohol | 10% |
| d) | Metallisches Jod als Sublimat | 4% |
| e) | Ein Monoglycerid einer polyoxiethylenisierten Fettsäure | 10% |
| f) | Propantriol | 5% |
| g) | Hexylenglykol | 16% |
| h) | Phosphorsäure in ausreichender Menge, um den pH-Wert des Präparates auf 4,5 einzustellen; | |
wobei sämtliche Prozentangaben sich auf den Massenanteil des Wirkstoffs am Gesamtgewicht des antiviralen Gesamtprodukts beziehen.

## Revendications

1. Une composition pharmaceutique pour traiter l'infection causée par un rétrovirus, comprenant :
(i) une quantité efficace du point de vue thérapeutique d'une composition antivirale comprenant :
a) au moins un iodure d'alkyldiméthylbenzylammonium de formule (I) 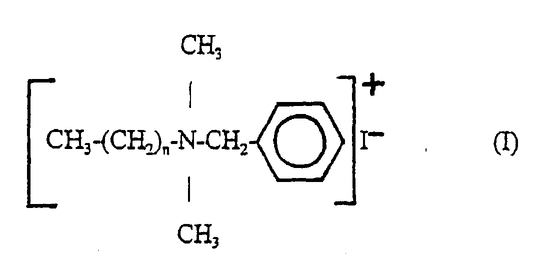 dans laquelle n est un nombre entier variant entre 10 et 20, avec une proportion en poids, par rapport à la composition antivirale totale comprise entre 40 et 50 %.
b) au moins un iodure d'ammonium quaternaire et un amide ayant la formule générale (II) 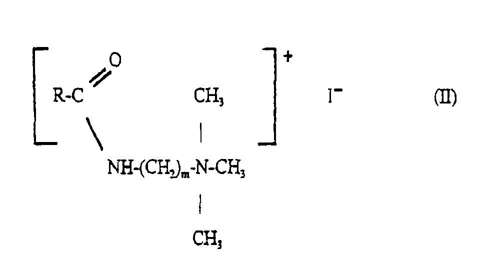 dans laquelle R est un groupe alkyle possédant jusqu'à 20 atomes de carbone ou groupe alkényle possédant jusqu'à 20 atomes de carbones et m est un nombre entier variant de 1 à 10, avec une proportion en poids, par rapport à la composition antivirale totale, comprise entre 5 et 15 %.
c) au moins un alcool gras oxyéthyléné, avec 8 masses d'oxyde d'éthylène, avec une proportion en poids par rapport à la composition antivirale totale comprise entre 5 et 15 %.
d) De l'iode métallique sublimé, avec une proportion en poids, par rapport à la composition antivirale totale comprise entre 2 et 6 %.
e) Un monoglycéride d'un acide gras polyoxyéthyléné avec une proportion en poids, par rapport à la composition antivirale totale, comprise entre 5 et 15 %.
f) Du propanotriol avec une composition en poids, par rapport à la composition antivirale totale, comprise entre 3 et 7 %.
g) De l'hexilenglycol avec une proportion en poids, par rapport à la composition antivirale totale comprise entre 10 et 20 % et
h) Au moins un acide organique dans une quantité suffisante pour adapter la valeur de pH de la composition de 4 à 6 et
(ii) un véhicule acceptable du point de vue pharmaceutique.

2. Composition pharmaceutique d'après la revendication 1, conçue pour désactiver ou diminuer la puissance infectieuse du virus de l'immunodéficience humaine (VIH).

3. Composition pharmaceutique d'après la revendication 2, conçue pour traiter le Syndrome de l'Immunodéficience Acquise (SIDA).

4. Composition pharmaceutique d'après l'une quelconque des revendications 1 à 3, comprenant :
(i) une quantité efficace du point de vue thérapeutique d'une composition antivirale comprenant :
| | | |
|---|---|---|
| a) | du iodure de diméthylstéarilbenzylammonium | 45 % |
| b) | du iodure de N-1-3 (3-triméthylammonium)-propyle-undécilénamide | 10 % |
| c) | un alcool gras oxyéthyléné avec 8 masses d'oxyde d'éthylène | 10 % |
| d) | de l'iode métallique sublimé | 4 % |
| e) | un monoglycéride d'un acide gras polyoxyéthyléné | 10 % |
| f) | du propanotriol | 5 % |
| g) | de l'hexilenglycol | 16 % |
| h) | de l'acide phosphorique dans une quantité suffisante pour adapter la valeur de pH de la composition à 4,5 ; toutes les proportions sont exprimées en termes de pourcentages par rapport au poids total de la composition antivirale et | |
(ii) un véhicule acceptable du point de vue pharmaceutique.

5. Utilisation d'une composition antivirale formée par :
a) au moins un iodure d'alkyldiméthylbenzylammonium de formule (I) 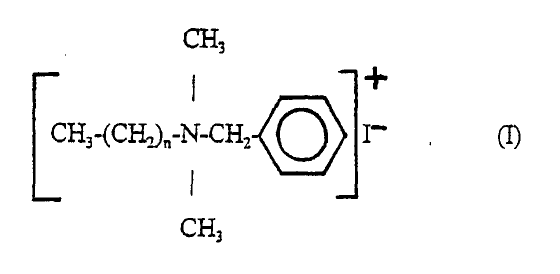 dans laquelle n est un nombre entier variant de 10 à 20 ; avec une proportion en poids, par rapport à la composition antivirale totale comprise entre 40 et 50 %.
b) au moins un iodure d'ammonium quaternaire et un amide ayant la formule générale (II) 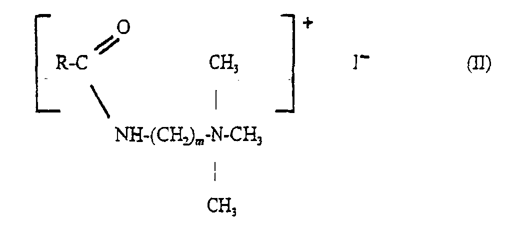 dans laquelle R est un groupe alkyle qui possède jusqu'à 20 atomes de carbone ou un groupe alkényle qui possède jusqu'à 20 atomes et m est un nombre entier qui varie de 1 à 10, avec une proportion en poids, par rapport à la composition antivirale totale, comprise entre 5 et 15 %.
c) au moins un alcool gras oxyéthyléné avec 8 masses d'oxyde d'éthylène, avec une proportion en poids, par rapport à la composition antivirale totale, comprise entre 2 et 15 %.
d) De l'iode métallique sublimé, avec une proportion en poids, par rapport à la composition antivirale totale, comprise entre 2 et 6 %.
e) Un monoglycéride d'un acide gras polyoxyéthyléné avec une proportion en poids, par rapport à la composition antivirale totale, comprise entre 5 et 15 %.
f) Du propanotriol avec une proportion en poids, par rapport à la composition antivirale totale comprise entre 3 et 7 %.
g) De l'hexilenglycol avec une proportion en poids, par rapport à la composition antivirale totale comprise entre 10 et 20 % ;
h) Au moins un acide organique dans une quantité suffisante pour adapter la valeur de pH de la composition de 4 à 6 en vue de préparer un médicament pour le traitement d'infections provoquées par des rétrovirus.

6. Utilisation d'après la revendication 5, selon laquelle ledit médicament convient pour désactiver ou réduire la puissance infectieuse du virus de l'immunodéficience humaine (HIV).

7. Utilisation d'après la revendication 6 dans laquelle ledit médicament convient pour le traitement du Syndrome de l'Immunodéficience Acquise (SIDA).

8. Utilisation d'après l'une quelconque des revendications 5 à 7 dans laquelle la composition antivirale est formée par :
| | | |
|---|---|---|
| a) | du iodure de diméthylstéarilbenzylammonium | 45 % |
| b) | du iodure de N-1-(triméthylammonium)-propyle-undécilénamide | 10 % |
| c) | un alcool gras oxyéthyléné avec 8 masses d'oxyde d'éthylène | 10 % |
| d) | de l'iode métallique sublimé | 4 % |
| e) | un monoglycéride d'un acide gras polyoxyéthiléné | 10 % |
| f) | du propanotriol | 5 % |
| g) | de l'hexylenglycol | 16 % |
| h) | de l'acide phosphorique dans une quantité suffisante pour adapter la valeur de pH de la composition à 4,5 ; toutes les proportions sont exprimées en termes de pourcentages par rapport au poids total de la composition antivirale. | |
